# EUROPÄISCHE PATENTSCHRIFT

(11) **EP 1 243 255 B1**
(45) Veröffentlichungstag und Bekanntmachung des Hinweises auf die Patenterteilung: **07.12.2005**
(21) Anmeldenummer: 02005025.8
(22) Anmeldetag: 06.03.2002
(51) Int. Cl.: A61K 9/16, A61K 9/14, A61K 31/65, A61K 31/7036, A61K 47/20, A61P 31/04

(54) **Verfahren zur Herstellung von antibiotischen Kompositen**
Method of preparation of antibiotic compositions
Procédé de préparation de compositions antibiotiques

(30) Priorität: 22.03.2001 DE 10114364
(43) Veröffentlichungstag der Anmeldung: 25.09.2002
(73) Patentinhaber: Heraeus Kulzer GmbH, 63450 Hanau (DE)
(72) Erfinder: Vogt, Sebastian F., Dr., 07749 Jena (DE); Schnabelrauch, Matthias, Dr., 07745 Jena (DE); Kühn, Klaus-Dieter, Dr., 35041 Marburg (DE)
(74) Vertreter: Kühn, Hans-Christian

(56) Entgegenhaltungen:
- DE-A- 3 248 328
- GB-A- 1 046 332
- GB-A- 1 478 240
- US-A- 3 022 286
- US-A- 3 536 759
- US-A- 5 607 685
- US-A- 6 077 822

## Beschreibung

Die vorliegende Erfindung betrifft ein Verfahren zur Herstellung von antibiotischen Kompositen, die als Implantate für die Human- und Veterinärmedizin zur Behandlung lokaler mikrobieller Infektionen im Hart- und im Weichgewebe eingesetzt werden können.

Es ist seit langem bekannt, daß eine systemische Anwendung von Antibiotika mit einer Reihe von Problemen behaftet ist. Bei der systemischen Anwendung ist es oft erforderlich, sehr hohe Antibiotika-Dosen einzusetzen, damit im infizierten Gewebe antimikrobiell wirksame Antibiotika-Konzentrationen erzielt werden. Dadurch kann es insbesondere bei den Aminoglykosid-Antibiotika und bei den Antibiotika vom Tetracyclin-Typ auf Grund ihrer Nephro- und Ototoxizität zu schwerwiegenden Schädigungen des Organismus kommen. Daher wurde schon seit Jahrzehnten die Idee verfolgt, Antibiotika in lokal applizierbaren Freisetzungssystemen einzusetzen, bzw. sie in geeignete Depotformen zu überführen. Für die Behandlung lokaler mikrobieller Infektionen des Weich- und Hartgewebes in der Human- und Veterinärmedizin ist es insbesondere wichtig, daß nach einer initial hohen Antibiotika-Dosis eine Freisetzung von geringen Antibiotika-Mengen über einen Zeitraum von Tagen bis zu mehreren Wochen gewährleistet wird, um eine möglichst weitgehende Abtötung der Mikroorganismen zu erreichen. Das ist vor allem bei Infektionen des Knochengewebes von entscheidender Bedeutung für eine erfolgreiche Infektionsbekämpfung. Besonders interessant sind hierbei solche Komposite, die neben einer antibiotischen Wirkung auch auf Grund ihrer chemischen Zusammensetzung und ihrer Struktur eine osteokonduktive Wirksamkeit zeigen.

Für die medizinische Anwendung von antibiotischen Depotsystemen ist es erforderlich, die entsprechenden Antibiotika mit geeigneten Hilfsstoffen zu handhabbaren und lagerfähigen Pharmaka bzw. Implantaten zu formulieren. Diese Pharmaka und Implantate stellen Kompositsysteme aus dem jeweiligen Wirkstoff und den zur Formulierung erforderlichen Hilfsstoffen dar. Diese Formulierungen können sich sowohl im flüssigen oder im festen Aggregatzustand befinden. Feste Formulierungen in Form von Formkörpern, Tabletten, Granulaten und Pulvern erfordern eine hinreichende mechanische Stabilität. Dazu ist es notwendig, den Wirkstoff und die Hilfsstoffe miteinander mechanisch stabil zu verbinden. Das kann einerseits durch chemische Aushärtungsprozesse der Hilfsstoffe erfolgen und andererseits durch Verpressung von plastisch verformbaren Hilfsstoffen, die unter Druckeinwirkung einen Verbund bilden.

Antibiotische Depotsysteme für die Behandlung von lokalen Infektionen sind Gegenstand einer Vielzahl von Veröffentlichungen und Patenten, von denen hier nur einige exemplarisch referiert werden können

Die physikalische Fixierung von Antibiotika unter Verwendung von nichtresorbierbaren Kunststoffen war Inhalt einer Reihe von Patenten, von denen hier nur einige aufgeführt werden. So schlägt Klemm (K. Klemm: Surgical synthetic-resin material and method of treating osteomyelitis. 13.05.1975, **US 3,882,858**) die Behandlung von Osteomyelitis mit Kunststoffpartikeln aus Polymethacrylat, Polyacrylat sowie deren Kopolymeren vor, die mit Gentamicin oder anderen Antibiotika beladen sind. Klemm beschreibt die Anwendung von Septopal (K. Klemm: Septopal - a new way of local antibiotic therapy. In T. J. G. Van Rens, F. H. Kayser (Eds.), Local antibiotic Treatment in Osteomyelitis and Soft-Tissue Infections, Excerpta Medica, Amsterdam (1981) 24-31; K. Klemm: Antibiotic beat chains. Clin. Orthop. Relat. Res. 295 (1993) 63-76.). Hierbei handelt es sich um kommerziell verfügbare Gentamicin-freisetzende Ketten aus Polymethacrylat. Heuser und Dingeldein beschreiben eine Komposition auf der Basis von Antibiotika und Polymethymethacrylat bzw. Polyacrylat, der als zusätzliche Komponente Aminosäuren zugefügt sind (D. Heuser, E. Dingeldein: Synthetic resin-base, antibiotic compositions containing amino acids. 04.04.1980, **US 4,191,740**; D. Heuser, E. Dingeldein: Synthetic resin-base, antibiotic compositions containing amino acids. 11.11.1980, **US 4,233,287**). Weiterhin wurden auch Antibiotika, insbesondere Aminoglykosid-Antibiotika, in Knochenzemente integriert (A. Gross, R. Schaefer, S. Reiss: Bone cement compositions containing gentamicin. 22.11.1977, **US 4,059,684**; A. Welch: Antibiotics in acrylic bone cement. In vitro studies. J. Biomed. Mater. Res. 12 (1978) 679; R. A. Elson, A. E. Jephott, D. B. McGechie, D. Vereitas: Antibiotic-loaded acrylic cement. J. Bone Joint Surg. 59B (1977) 200-205). Als Bindemittel wirken hierbei die bei der Zementaushärtung entstehenden Polymere.

Die Depotbildung von Antibiotika mit Hilfe von resorbierbaren Kunststoffen, insbesondere von Polyestern der α-Hydroxycarbonsäuren, war ebenfalls Gegenstand einer Reihe von Publikationen, von denen hier ebenfalls nur einige beispielhaft referiert werden. Sampath et. al. schlagen ein Gentamicin-freisetzendes System bestehend aus Poly-L-lactid und Gentamicin vor, das durch Verpressung von Poly-L-lactid/Gentamicin-Mikrokapseln hergestellt wurde (S. S. Sampath, K. Garvin, D. H. Robinson: Preparation and characterization of biodegradable poly(-L-lactic acid) gentamicin delivery systems. Int. J. Pharmaceutics 78 (1992) 165-174). Dieses System zeigt in Abhängigkeit von der eingesetzten Menge an Gentamicin eine nicht unbeträchtliche Verzögerung der Wirkstofffreisetzung. Bei einem ähnlichen System wurde Poly-D,L-lactid zur Herstellung von Wirkstoff enthaltenden Mikrosphären genutzt (R. Bodmeier, J. W. McGinity: The preparation and evaluation of drug-containing poly(D,L-lactide) microspheres formed by solvent evaporation method. Pharm. Res. 4 (1987) 465-471). Von Friess und Schlapp werden ebenfalls Mikropartikel aus Polylactid beschrieben, die mit Kollagen/Gentamicinsulfat beschichtet sind (W. Friess, M. Schlapp: Advanced implants for local delivery of gentamicin. Sixth World Biomaterials Congress Transactions (2000) 1488). Diese beschichteten Mikrosphären zeigten nur eine sehr geringe Tendenz, die Gentamicin-Freisetzung zu verzögern. Von Schmidt et al. wurden Gentamicin enthaltende resorbierbare Formkörper vorgeschlagen (C. Schmidt, R. Wenz, B. Nies, F. Moll: Antibiotic in vivo/in vitro release, histocompatibility and biodegradation of gentamicin implants based on lactic acid polymers and copolymers. J. Control. Release 37 (1995) 83-94). Diese Körper wurden durch Verpressung von Gemischen aus Gentamicin-Sulfat/Poly-L-lactid, Gentamicin-Sulfat/Poly-D,L-lactid und Gentamicinsulfat/Poly-D,L-lactid-coglykolid hergestellt. Diese Depotpräparate setzten zirka neunzig Prozent des Antibiotikums innerhalb von vierundzwanzig Stunden frei.

Neben den auf Kunststoffen basierenden wurden auch zahlreiche anorganische Systeme mit retardierender Wirkung beschrieben. Im folgenden werden nur einige der mit Calciumsulfat hergestellten Systeme kurz referiert. So wird von Randolph et al. ein retardierendes System beschrieben, daß auf dem Einschluß von Wirkstoffen in eine Calciumsulfat-Matrix beruht (D. A. Randolph, J. L. Negri, T. R. Devine, S. Gitelis: Calcium sulfate controlled release matrix. 15.09.1998, **US 5,807,567**). Die Herstellung dieser Calciumsulfat-Pellets erfolgt dabei ausgehend von einem Gemisch aus α-Calciumsulfat-Hemihydrat, β-Calciumsulfat-Hemihydrat, einem Additiv und Wasser. Die Härtung erfolgt durch Bildung von Calciumsulfat-Dihydrat. Turner et al. beschreiben Tabletten aus Calciumsulfat, die Tobramycin enthalten und zur Behandlung von Medullar-Defekten Verwendung finden sollen (T. M. Turner, R. M. Urban, S. Gitelis, A. M. Lawrence-Smith, D. J. Hall: Delivery of tobramycin using calcium sulfate tablets to graft a large medullary defect: Local and systemic effects. Sixth World Biomaterials Congress Transactions (2000) 767.) Ähnliche Freisetzungssysteme aus Calciumsulfat, aber mit Amikacin-Sulfat, werden ebenfalls beschrieben (D. W. Petersen, W.O. Haggard, L. H. Morris, K. C. Richelsoph, J. E. Parr: Elution of amikacin from calcium sulfate pellets: An in vitro study. Sixth World Biomaterials Congress Transactions (2000) 767).

Bisher fanden schwerlösliche Salze der Aminoglykosid-Antibiotika und der Lincosamid-Antibiotika relativ geringe Beachtung für die Herstellung von Depotpräparaten. Die Bildung von schwerlöslichen Salzen bzw. Chelaten der Antibiotika des Tetracyclintyps ist seit Jahrzehnten allgemeiner Kenntnisstand. So beschreibt Folch Vazquez die Herstellung von Tetracyclindodecylsulfat durch Umsetzung von Tetracyclinhydrochlorid mit Natriumdodecylsulfat in Wasser (C. Folch Vazquez: Tetracycline lauryl sulfate. 08.02.1966, **ES 3 309 402**; C. Folch Vazquez: Tetracycline derivatives. 09.01.1967, **NL 6609490**). Alternativ kann die Herstellung auch ausgehend von Tetracyclin und der Dodecylschwefelsäure erfolgen (C. Folch Vazquez: Tetracycline lauryl sulfate. 08.02.1966, **ES 322 771**). Weiterhin wurde auch die Verwendung von Tetracyclin-Sulfamaten zur antibiotischen Therapie vorgeschlagen (A. Jurando, J. M. Puigmarti: Antibiotic tetracycline sulfamate and its derivatives. 27.10.1970, **US 3,536,759;** Anonym: Antibiotic tetracycline alkylsulfamates. 16.10.1969, **ES 354173;** C. Ciuro, A. Jurado: Stability of a tetracycline derivative. Afinidad 28 (292) 1971, 1333-5.). Bei den Aminoglykosid-Antibiotika sind ebenfalls eine Reihe von schwerlöslichen Salzen prinzipiell bekannt. So wurde beim Gentamicin die Darstellung schwerlöslicher Salze basierend auf höheren Fettsäuren, Arylalkylcarbonsäuren, Alkylsulfaten und Alkylsulfonaten beschrieben (G. M. Luedemann, M. J. Weinstein: Gentamycin and method of production. 16.07.1962, **US 3,091,572**). Exemplarisch sind dafür Gentamicin-Salze der Laurinsäure, der Stearinsäure, der Palmitinsäure, der Ölsäure, der Phenylbuttersäure, der Naphthalen-1-carbonsäure, der Laurylschwefelsäure und der Dodecylbenzensulfonsäure. Diese Salze erwiesen sich vielfach als unvorteilhaft, weil sie wachsartige, hydrophobe Substanzen darstellen, die eine galenische Verwendung behindern. Trotzdem wurden Festtsäuresalze von Gentamicin und von Etamycin aus der freien Base bzw. aus ihren Salzen in Wasser bei 50-80°C synthetisert (H. Voege, P. Stadler, H. J. Zeiler, S. Samaan, K. G. Metzger: Sparingly - soluble salts of aminoglycosides and formations containing them with inhibited substance-release. 28.12.1982 **DE 3 248 328**). Diese Antibiotika-Fettsäuresalze sollen als Injektionspräparate geeignet sein. Die Herstellung von Gentamicindodecylsulfat und dessen Verwendung in Salben, Cremes wurde ebenfalls beschrieben (C. Folch Vazquez: Gentamicin derivates. 29.10.1974, **BE 821 600**). Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, **US 4,617,293**). Es werden die Salze der Phosphorsäurehalbester von Derivaten der Hydroxyflavane, Hydroxyflavene, Hydroxyflavanone, Hydroxyflavone und Hydroxyflavylium beschrieben. Besonders bevorzugt sind die Derivate der Flavanone und der Flavone. Diese schwerlöslichen Salze sollen als Depotpräparate Verwendung finden. So werden zum Beispiel diese Salze in Kollagenvliese eingebracht (H. Wahlig, E. Dingeldein, D. Braun: Medicinally useful, shaped mass of collagen resorbable in the body. 22.09.1981, **US 4,291,013**). Weiterhin wurden auch künstliche Herzklappen mit diesen schwerlöslichen Gentamicin-Salzen, Gentamicin-Crobefat, imprägniert (M. Cimbollek, B. Nies, R. Wenz, J. Kreuter: Antibiotic-impregnated heart valve sewing rings for treatment and prophylaxis of bacterial endocarditis. Antimicrob. Agents Chemother. 40(6) (1996)1432-1437). Interessant ist bei dieser Veröffentlichung insbesondere, daß ein Gemisch aus leichtlöslichem Gentamicin-Sulfat und schwerlöslichem Gentamicin-Crobefat eingesetzt wird. Das Ziel war dabei, daß einerseits nach Einbringung der Herzklappenringe in den Organismus bzw. in eine Modellflüssigkeit eine hohe initiale Gentamicin-Konzentration durch das leichtlösliche Gentamicin-Sulfat erreicht wird und das andererseits durch das relativ schwerlösliche Gentamicin-Crobefat eine Freisetzung von Gentamicin über einen längeren Zeitraum möglich wird.

Der vorliegenden Erfindung liegt die Aufgabe zugrunde, ein Verfahren zur Herstellung von antibiotischen Kompositen zu entwickeln, daß eine einfache kostengünstige Fertigung von Kompositen zuläßt, ohne daß anorganische oder organische, nichtantibiotische Bindemittel notwendig sind. Diese antibiotischen Komposite sollen als Implantate im Bereich der Human- und Veterinärmedizin, zur Behandlung lokaler mikrobieller Infektionen im Knochen- und im Weichgewebe eingesetzt werden können. Weiterhin soll das zu entwickelnde Verfahren nicht nur für ein spezielles Antibiotikum anwendbar sein, sondern es sollte sich vielmehr für eine Reihe von Antibiotika ähnlicher Struktur eignen.

Die Erfindung beruht auf dem überraschenden Befund, daß die an sich bekannten organischen Sulfate, organischen Sulfonate und aliphatischen Carboxylate der Antibiotika vom Aminoglykosid-Typ, dem Lincosamid-Typ und dem Tetracyclin-Typ, die im allgemeinen hydrophobe, wachsartige Substanzen darstellen, plastisch verformbar sind und Bindemitteleigenschaften aufweisen. Es zeigte sich, daß diese plastisch verformbaren Salze unter Druckeinwirkung mit Hilfsstoffen feste Verbunde bilden. Dadurch ist es möglich, diese plastisch verformbaren Antibiotika-Salze als Bindemittel bei der Herstellung von antibiotischen Kompositen aus anorganischen Materialien und ggf. organischen Materialien einzusetzen. Zusätzliche Bindemittel zur Gewährleistung der Formstabilität der Komposite sind nicht mehr notwendig. Dadurch können Kosten eingespart werden und es gibt keine eventuellen Probleme mit der Biokompatibilität und der Resorbierbarkeit von zusätzlichen anorganischen und/oder organischen Bindemitteln. Der besondere Vorteil des erfindungsgemäßen Bindemittels zur Herstellung von antibiotischen Kompositen, die zur lokalen Infektionsbekämpfung Verwendung finden sollen, besteht darin, daß nach der Einbringung der erfindungsgemäß hergestellten Komposite in wässriges Mileu eine Auflösung des Bindemittels unter Antibiotika-Freisetzung einsetzt, wobei mit zunehmender Auflösung des Bindemittels ein zeitgleicher Zerfall der Komposite stattfindet. Das bedeutet, mit zunehmender Antibiotika-Freisetzung nimmt auch der Zerfall der Komposite zu.

Die Erfindung beruht weiterhin auf dem überraschenden Befund, daß die an sich bekannten organischen Sulfate und organischen Sulfonate der Antibiotika vom Aminoglykosid-Typ, dem Lincosamid-Typ und dem Tetracyclin-Typ in Gegenwart von anorganischen Kompositkomponenten und ggf. organischen Kompositkomponenten durch Einwirkung von Wasser während der Formgebung der Formkörper aus handelüblichen, wasserlöslichen Antibiotika-Salzformen, wie zum Beispiel den Sulfaten, durch Umsetzung mit in Wasser löslichen organischen Sulfaten und Sulfonaten gebildet werden. Durch diese in situ Bildung der plastisch verformbaren Salze, ist eine separate Synthese der Salze nicht mehr notwendig. Dadurch können kostenintensive Synthese- und Reinigungsschritte eingespart werden.

Erfindungsgemäß ist, daß ein plastisch verformbares Salz, das aus mindestens einer kationischen Komponente einer protonierten Antibiotika-Base aus den Gruppen der Aminoglykosid-Antibiotika, der Lincosamid-Antibiotika und der Tetracyclin-Antibiotika und aus mindestens einer anionischen Komponente aus der Gruppe der aliphatischen Carboxylate, Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate aufgebaut ist, als Bindemittel (zur Fixierung von anorganischen Kompositbestandteilen und/oder ggf. organischen Kompositbestandteilen und) ggf. unter Beimischung von Wasser zur Formgebung der Komposite insbesondere durch Pressen, Strangpressen, Walzen, Kalandrieren und Mahlen verwendet wird.

Die nachfolgenden Ausführungsformen haben sich in der Praxis bewährt.

Weiterhin ist erfindungsgemäß, daß als anionische Komponente aus der Gruppe der Alkylsulfate besonders Dodecylsulfat, Tetradecylsulfat, Hexadecylsulfat, Octadecylsulfonat und Docosanolsulfat verwendet werden.

Erfindungsgemäß ist, daß als anionische Komponente aus der Gruppe der Alkylsulfonate Dodecylsulfonat, Hexadecylsulfonat und Octadecylsulfonat bevorzugt werden.

Es ist auch erfindungsgemäß, daß als anionische Komponente aliphatische Carboxylate verwendet werden, die 12 bis 30 Kohlenstoffatome enthalten.

Erfindungsgemäß ist, daß als anionische Komponente aus der Gruppe der aliphatischen Carboxylate Palmitat, Stearat und Behenylat verwendet werden.

Erfindungsgemäß ist, daß das plastisch verformbare Salz vor dem Formgebungsprozeß synthetisiert wird.

Es ist erfindungsgemäß, daß das plastisch verformbare Salz während der Formgebung der Komposite durch Einbringung von Wasser in ein Gemisch bestehend aus anorganischen Kompositbestandteilen, ggf. organischen Kompositbestandteilen, einem oder mehreren Vertretern der Aminoglykosid-Antibiotika und/oder der Lincosamid-Antibiotika und/oder der Tetracyclin-Antibiotika, die in der Sulfat-Form, der Hydrochlorid-Form, Hydrobromid-Form und Phosphat-Form vorliegen, und einem oder mehreren Vertretern der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate, die in der Natriumsalzform und/oder in der Kaliumsalzform und/oder in der Ammoniumsalzform und/oder in der Trialkylammoniumsalzform und/oder in der Dialkylammoniumsalzform und/oder der Monoalkylammoniumsalzform und/oder in der Triarylammoniumsalzform und/oder in der Diarylammoniumsalzform und/oder in der Arylammoniumsalzform und/oder in der Alkyldiarylammoniumsalzform und/oder in der Dialkylarylammoniumsalzform und/oder der Tricycloalkylammoniumsalzform und/oder der Dicycloalkylammoniumsalzform und/oder der Monocycloalkylammoniumsalzform und/oder der Alkyldicycloalkytammoniumsalzform und/oder in der Dialkylcycloalkylammoniumsalzform vorliegen, gebildet wird.

Erfindungsgemäß ist, daß die Alkylsulfate, Arylsulfate, Alkylarylsulfate, Cycloalkylsulfate und Alkylcycloalkylsulfate Schwefelsäurehalbester sind.

Erfindungsgemäß ist auch, daß als Alkylsulfate Natriumdodecylsulfat, Natriumtetradecylsulfat, Natriumhexadecylsulfat und Natriumoctadecylsulfat bevorzugt sind.

Erfindungsgemäß ist, daß als Alkylsulfonate besonders Natriumdodecylsulfonat, Natriumhexadecylsulfonat und Natriumoctadecylsulfonat bevorzugt sind.

Weiterhin ist erfindungsgemäß, daß als Alkylarylsulfonat besonders Natriumdodecylbenzylsulfonat bevorzugt wird.

Erfindungsgemäß ist, daß Allomycin, Amicetin, Amikacin, Apramycin, Bekanamycin, Betamicin, Butirosin, Destomycin, Dibekacin, Dihydrostreptomycin, Flambamycin, Fortimycin A, Fortimycin B, Framycetin, Gentamicin, Hikizimycin, Homomycin, Hybrimycin, Hygromycin B, Kanamycin, Kasuhamycin, Lividomycin, Minosaminoycin, Neomycin, Netilmicin, Paromomycin, Parvulomycin, Puromycin A, Ribostamycin, Rimocidin, Ristosamin, Ristomycin, Sagamycin, Sisomicin, Sorbistin, Spectinomycin, Streptomycin, Tobramycin, Tunicamycin, Verdamycin aus der Gruppe der Aminoglykosid-Antibiotika bevorzugt werden.

Erfindungsgemäß ist, daß Clindamycin und Lincomycin aus der Gruppe der Lincosamid-Antibiotika bevorzugt werden.

Erfindungsgemäß ist, daß Tetracyclin, Chlortetracyclin, Oxytetracylin, Demethylchlortetracyclin, Methacyclin, Doxycyclin, Rolitetracyclin und Minocyclin aus der Gruppe der Tetracyclin-Antibiotika bevorzugt werden.

Es ist auch erfindungsgemäß, daß als anorganische Kompositbestandteile bevorzugt Calciumcarbonat, Magnesiumcarbonat, Calciumhydroxid, Magnesiumhydroxid, Magnesiumoxid, Calciumsulfat, Calciumsulfat-Hemihydrat, Calciumsulfat-Dihydrat, Tricalciumphosphat, Tetracalciumphosphat, Calciumhydrogenphosphat, Calciumhydrogenphosphat-Dihydrat, Hydroxylapatit, Fluorapatit, resorbierbares Glas, resorbierbare Glaskeramik und deren Gemischen verwendet werden.

Weiterhin ist erfindungsgemäß, daß die anorganischen Kompositbestandteile in Form von Pulvern und/oder Granulaten verwendet werden.

Erfindungsgemäß ist es ferner, daß als organische Kompositbestandteile bevorzugt Stärke, Cellulose, Chitin, Chitosan, Gelatine, Kollagen, Polymethacrylsäureester, Polyacrylsäurester, Polyvinylalkohol, Polyvinylchlorid, Polyvinylidenchlorid und Polytetrafluorethylen und deren Gemische verwendet werden.

Erfindungsgemäß ist, daß in Wasser leicht lösliche Antibiotika als organische Kompositkomponente verwendet werden.

Es ist auch erfindungsgemäß, daß in den Kompositen der Massenanteil des plastisch verformbaren Salzes 0,1 bis 98 Masseprozent beträgt.

Erfindungsgemäß ist, daß die Komposite zu Formkörpern, Granulaten und Pulvern geformt werden.

Erfindungsgemäß ist, daß die Komposite plastisch verformbar sind.

Im Sinne der Erfindung ist es ebenfalls, daß die Komposite in Form von Pasten bevorzugt sind. Damit ist es möglich die Komposite zu kneten und in infizierte Hartgewebedefekte einzumodellieren.

Erfindungsgemäß ist weiterhin, daß die Komposite als Beschichtung auf resorbierbare Implantate und auf nichtresorbierbare Implantate aufgebracht werden

Der Gegenstand der vorliegenden Erfindung soll anhand der nachfolgenden Beispiele 1 bis 2 näher erläutert werden.

### Herstellung der Antibiotikazubereitungen

### Beispiel 1:

Es wird ein Gemisch aus 25 mg Gentamicinsulfat (700 U/mg, Fluka), 50 mg Gentamicinpentakis-dodecylsulfat und 1425 mg Calciumsulfat-Dihydrat (Fluka) durch Vermahlen hergestellt. Jeweils 200 mg dieses Gemischs werden mit einer Presse bei einem Druck von 5 Tonnen innerhalb von zwei Minuten zu scheibenförmigen, stabilen Formkörpern, mit einem Durchmesser von 13 mm, gepreßt.

### Beispiel 2:

Es wird ein Gemisch aus 25 mg Gentamicinsulfat (700 U/mg, Fluka), 48 mg Gentamicinpentakis-dodecylsulfonat und 1427 mg Calciumhydrogenphosphat (Fluka) durch Vermahlen hergestellt. Jeweils 200 mg dieses Gemischs werden mit einer Presse bei einem Druck von 5 Tonnen innerhalb von zwei Minuten zu scheibenförmigen, stabilen Formkörpern, mit einem Durchmesser von 13 mm, gepreßt.

### Antibiotika-Freisetzungsversuche

Die in den Beispielen **1** und **2** hergestellten Formkörper wurden in physiologische Kochsalzlösung eingebracht und in dieser bei 37°C über einen Zeitraum von zwölf Tagen gelagert, um die retardierte Antibiotika-Freisetzung zu bestimmen. Die Probennahme erfolgte nach 1,3,6,9 und 12 Tagen Lagerungszeit. Die Antibiotika-Wertbestimmung wurde mit einem Agardiffusionstest unter Verwendung von Bacillus subtilis ATCC 6633 als Testkeim durchgeführt (Ergebnisse siehe **Tab.1**).

**Tab.1:**

| Kumulierte Gentamicin-Freisetzung aus Probekörpern der **Beispiele 1** und **2** in Abhängigkeit von der Lagerungszeit in physiologischer Kochsalzlösung bei 37°C. | | | | | |
|---|---|---|---|---|---|
| Beispiel | Kumulierte Gentamicin-Freisetzung [Ma%] | | | | |
| | Lagerungszeit [d] | | | | |
| | **1** | 3 | 6 | 9 | 12 |
| **1** | 73 | 84 | 90 | 95 | 100 |
| **2** | 55 | 76 | 87 | 97 | 100 |

## Patentansprüche

1. Verfahren zur Herstellung von antibiotischen Kompositen, **dadurch gekennzeichnet, dass** ein plastisch verformbares Salz, das aus mindestens einer kationischen Komponente einer protonierten Antibiotika-Base aus den Gruppen der Aminoglykosid-Antibiotika, der Lincosamid-Antibiotika und der Tetracyclin-Antibiotika und aus mindestens einer anionischen Komponente aus der Gruppe der aliphatischen Carboxylate, Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate aufgebaut ist, als Bindemittel ggf. unter Beimischung von Wasser zur Formgebung von Kompositen verwendet wird.

2. Verfahren zur Herstellung von antibiotischen Kompositen nach Anspruch 1, **dadurch gekennzeichnet, dass** die Formgebung mittels Pressen und/oder Strangpressen und/oder Walzen und/oder Kalandrieren und/oder Mahlen bewerkstelligt wird.

3. Verfahren zur Herstellung von antibiotischen Kompositen nach einem der Ansprüche 1 bis 2, **dadurch gekennzeichnet, dass** als anionische Komponente aus der Gruppe der Alkylsulfate besonders Dodecylsulfat, Tetradecylsulfat, Hexadecylsulfat, Octadecylsulfonat und Docosanolsulfat verwendet werden.

4. Verfahren zur Herstellung von antibiotischen Kompositen nach einem der Ansprüche 1 bis 2, **dadurch gekennzeichnet, dass** als anionische Komponente aus der Gruppe der Alkylsulfonate Dodecylsulfonat, Hexadecylsulfonat und Octadecylsulfonat verwendet werden.

5. Verfahren zur Herstellung von antibiotischen Kompositen nach einem der Ansprüche 1 bis 2, **dadurch gekennzeichnet, dass** als anionische Komponente besonders aliphatische Carboxylate verwendet werden, die 12 bis 30 Kohlenstoffatome enthalten.

6. Verfahren zur Herstellung von antibiotischen Kompositen nach einem der Ansprüche 1 bis 2, **dadurch gekennzeichnet, dass** als anionische Komponente aus der Gruppe der aliphatischen Carboxylate Palmitat, Stearat und Behenylat verwendet werden.

7. Verfahren zur Herstellung von antibiotischen Kompositen nach einem der Ansprüche 1 bis 6, **dadurch gekennzeichnet, dass** das plastisch verformbare Salz vor dem Formgebungsprozess synthetisiert wird.

8. Verfahren zur Herstellung von antibiotischen Kompositen nach Anspruch 1, **dadurch gekennzeichnet, dass** das plastisch verformbare Salz während der Formgebung der Komposite durch Einbringung von Wasser in ein Gemisch bestehend aus anorganischen Kompositbestandteilen, ggf. organischen Kompositbestandteilen, einem oder mehreren Vertretern der Aminoglykosid-Antibiotika und/oder der Lincosamid-Antibiotika und/oder der Tetracyclin-Antibiotika, die in der Sulfat-Form, der Hydrochlorid-Form, Hydrobromid-Form und Phosphat-Form vorliegen, und einem oder mehreren Vertretern der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate, die in der Natriumsalzform und/oder in der Kaliumsalzform und/oder in der Ammoniumsalzform und/oder in der Trialkylammoniumsalzform und/oder in der Dialkylammoniumsalzform und/oder der Monoalkylammoniumsalzform und/oder in der Triarylammoniumsalzform und/oder in der Diarylammoniumsalzform und/oder in der Arylammoniumsalzform und/oder in der Alkyldiarylammoniumsalzform und/oder in der Dialkylarylammoniumsalzform und/oder der Tricycloalkylammoniumsalzform und/oder der Dicycloalkylammoniumsalzform und/oder der Monocycloalkylammoniumsalzform und/oder der Alkyldicycloalkylammoniumsalzform und/oder in der Dialkylcycloalkylammoniumsalzform vorliegen, gebildet wird.

9. Verfahren zur Herstellung von antibiotischen Kompositen nach einem der Ansprüche 1 bis 2 oder 8, **dadurch gekennzeichnet, dass** die Alkylsulfate, Arylsulfate, Alkylarylsulfate, Cycloalkylsulfate und Alkylcycloalkylsulfate Schwefelsäurehalbester sind.

10. Verfahren zur Herstellung von antibiotischen Kompositen nach einem der Ansprüche 1, 2, 8 oder 9, **dadurch gekennzeichnet, dass** als Alkylsulfate Natriumdodecylsulfat, Natriumtetradecylsulfat, Natriumhexadecylsulfat und Natriumoctadecylsulfat verwendet werden.

11. Verfahren zur Herstellung von antibiotischen Kompositen nach einem der Ansprüche 1 bis 2 oder 8, **dadurch gekennzeichnet, dass** als Alkylsulfonate Natriumdodecylsulfonat, Natriumhexadecylsulfonat und Natriumoctadecylsulfonat verwendet werden.

12. Verfahren zur Herstellung von antibiotischen Kompositen nach einem der Ansprüche 1 bis 2 oder 8, **dadurch gekennzeichnet, dass** als Alkylarylsulfonat besonders Natriumdodecylbenzylsulfonat verwendet wird.

13. Verfahren zur Herstellung von antibiotischen Kompositen nach einem der Ansprüche 1 bis 12, **dadurch gekennzeichnet, dass** als anorganische Kompositbestandteile Calciumcarbonat, Magnesiumcarbonat, Calciumhydroxid, Magnesiumhydroxid, Magnesiumoxid, Calciumsulfat, Calciumsulfat-Hemihydrat, Calciumsulfat-Dihydrat, Tricalciumphosphat, Tetracalciumphosphat, Calciumhydrogenphosphat, Calciumhydrogenphosphat-Dihydrat, Hydroxylapatit, Fluorapatit, resorbierbares Glas, resorbierbare Glaskeramik und deren Gemische verwendet werden.

14. Verfahren zur Herstellung von antibiotischen Kompositen nach einem der Ansprüche 1 bis 13, **dadurch gekennzeichnet, dass** die anorganischen Kompositbestandteile in Form von Pulvern und/oder Granulaten verwendet werden.

15. Verfahren zur Herstellung von antibiotischen Kompositen nach einem der Ansprüche 1 bis 14, **dadurch gekennzeichnet, dass** als organische Kompositbestandteile bevorzugt Stärke, Cellulose, Chitin, Chitosan, Gelatine, Kollagen, Polymethacrylsäureester, Polyacrylsäurester, Polyvinylalkohol, Polyvinylchlorid, Polyvinylidenchlorid, Polytetrafluorethylen, Bienenwachs, Carnaubawachs, Triglyceride und deren Gemische verwendet werden.

16. Verfahren zur Herstellung von antibiotischen Kompositen nach einem der Ansprüche 1 bis 15, **dadurch gekennzeichnet, dass** ggf. in Wasser leicht lösliche Antibiotika als organische Kompositkomponente verwendet werden.

17. Verfahren zur Herstellung von antibiotischen Kompositen nach einem der Ansprüche 1 bis 16, **dadurch gekennzeichnet, dass** in den Kompositen der Massenanteil des plastisch verformbaren Salzes 0,1 bis 98 Masseprozent beträgt.

18. Verfahren zur Herstellung von antibiotischen Kompositen nach einem der Ansprüche 1 bis 17, **dadurch gekennzeichnet, dass** die Komposite zu Formkörpern, Granulaten und Pulvern geformt werden.

19. Verfahren zur Herstellung von antibiotischen Kompositen nach einem der Ansprüche 1 bis 18, **dadurch gekennzeichnet, dass** die Komposite plastisch verformbar sind.

20. Verfahren zur Herstellung von antibiotischen Kompositen nach einem der Ansprüche 1 bis 17 oder 19, **dadurch gekennzeichnet, dass** die Komposite in Form von Pasten verwendet werden.

21. Verfahren zur Herstellung von antibiotischen Kompositen nach einem der Ansprüche 1 bis 20, **dadurch gekennzeichnet, dass** die Komposite als Beschichtung auf resorbierbare Implantate und auf nichtresorbierbare Implantate aufgebracht werden.

## Claims

1. Process for the preparation of antibiotic composites, **characterized in that** a plastically deformable salt which is composed of at least one cationic component of a protonated antibiotic base from the group consisting of the aminoglycoside antibiotics, of the lincosamide antibiotics and of the tetracycline antibiotics, and of at least one anionic component from the group consisting of the aliphatic carboxylates, alkylsulphates, arylsulphates, alkylarylsulphates, cycloalkylsulphates, alkylcycloalkylsulphates, alkylsulphamates, cycloalkylsulphamates, alkylcycloalkylsulphamates, arylsulphamates, alkylarylsulphamates, alkylsulphonates, fatty acid-2-sulphonates, arylsulphonates, alkylarylsulphonates, cycloalkylsulphonates, alkylcycloalkylsulphonates, alkyldisulphates, cycloalkyldisulphates, alkyldisulphonates, cycloalkyldisulphonates, aryldisulphonates, alkylaryldisulphonates, aryltrisulphonates and alkylaryltrisulphonates, is used as the binder, optionally with admixing of water for shaping of composites.

2. Process for the preparation of antibiotic composites according to Claim 1, **characterized in that** the shaping is effected by means of pressing and/or extrusion and/or rolling and/or calendering and/or milling.

3. Process for the preparation of antibiotic composites according to either of Claims 1 and 2, **characterized in that** in particular dodecylsulphate, tetradecylsulphate, hexadecylsulphate, octadecylsulphonate and docosanylsulphate are used as the anionic component from the group consisting of the alkylsulphates.

4. Process for the preparation of antibiotic composites according to either of Claims 1 and 2, **characterized in that** dodecylsulphonate, hexadecylsulphonate and octadecylsulphonate are used as the anionic component from the group consisting of the alkylsulphonates.

5. Process for the preparation of antibiotic composites according to either of Claims 1 and 2, **characterized in that** in particular aliphatic carboxylates which contain 12 to 30 carbon atoms are used as the anionic component.

6. Process for the preparation of antibiotic composites according to either of Claims 1 and 2, **characterized in that** palmitate, stearate and behenylate are used as the anionic component from the group consisting of the aliphatic carboxylates.

7. Process for the preparation of antibiotic composites according to any of Claims 1 to 6, **characterized in that** the plastically deformable salt is synthesized before the shaping process.

8. Process for the preparation of antibiotic composites according to Claim 1, **characterized in that** the plastically deformable salt is formed during the shaping of the composites by introducing water into a mixture consisting of inorganic composite components, optionally organic composite components, one or more members of the aminoglycoside antibiotics and/or of the lincosamide antibiotics and/or of the tetracycline antibiotics, which are present in the sulphate form, hydrochloride form, hydrobromide form and phosphate form, and one or more members of the alkylsulphates, arylsulphates, alkylarylsulphates, cycloalkylsulphates, alkylcycloalkylsulphates, alkylsulphamates, cycloalkylsulphamates, alkylcycloalkylsulphamates, arylsulphamates, alkylarylsulphamates, alkylsulphonates, fatty acid-2-sulphonates, arylsulphonates, alkylarylsulphonates, cycloalkylsulphonates, alkylcycloalkylsulphonates, alkyldisulphates, cycloalkyldisulphates, alkyldisulphonates, cycloalkyldisulphonates, aryldisulphonates, alkylaryldisulphonates, aryltrisulphonates and alkylaryltrisulphonates, which are present in the sodium salt form and/or in the potassium salt form and/or in the ammonium salt form and/or in the trialkylammonium salt form and/or in the dialkylammonium salt form and/or the monoalkylammonium salt form and/or in the triarylammonium salt form and/or in the diarylammonium salt form and/or in the arylammonium salt form and/or in the alkyldiarylammonium salt form and/or in the dialkylarylammonium salt form and/or the tricycloalkylammonium salt form and/or the dicycloalkylammonium salt form and/or the monocycloalkylammonium salt form and/or the alkyldicycloalkylammonium salt form and/or in the dialkylcycloalkyl ammonium salt form.

9. Process for the preparation of antibiotic composites according to any of Claims 1, 2 and B, **characterized in that** the alkylsulphates, arylsulphates, alkylarylsulphates, cycloalkylsulphates and alkylcycloalkylsulphates are monoesters of sulphuric acid.

10. Process for the preparation of antibiotic composites according to any of Claims 1, 2, 8 and 9, **characterized in that** sodium dodecylsulphate, sodium tetradecylsulphate, sodium hexadecylsulphate and sodium octadecylsulphate are used as alkylsulphates.

11. Process for the preparation of antibiotic composites according to any of Claims 1, 2 and 8, **characterized in that** sodium dodecylsulphonate, sodium hexadecylsulphonate and sodium octadecylsulphonate are used as alkylsulphonates.

12. Process for the preparation of antibiotic composites according to any of Claims 1, 2 and 8, **characterized in that** in particular sodium dodecylbenzenesulphonate is used as the alkylarylsulphonate.

13. Process for the preparation of antibiotic composites according to any of Claims 1 to 12, **characterized in that** calcium carbonate, magnesium carbonate, calcium hydroxide, magnesium hydroxide, magnesium oxide, calcium sulphate, calcium sulphate hemihydrate, calcium sulphate dihydrate, tricalcium phosphate, tetracalcium phosphate, calcium hydrogen phosphate, calcium hydrogen phosphate dihydrate, hydroxylapatite, fluoroapatite, resorbable glass, resorbable glass ceramic and mixtures thereof are used as inorganic composite components.

14. Process for the preparation of antibiotic composites according to any of claims 1 to 13, **characterized in that** the inorganic composite components are used in the form of powders and/or granules.

15. Process for the preparation of antibiotic composites according to any of Claims 1 to 14, **characterized in that** starch, cellulose, chitin, chitosan, gelatin, collagen, polymethacrylic esters, polyacrylic esters, polyvinyl alcohol, polyvinyl chloride, polyvinylidene chloride, polytetrafluoroethylene, beeswax, carnauba wax, triglycides and mixtures thereof are used as organic composite components,

16. Process for the preparation of antibiotic composites according to any of Claims 1 to 15, **characterized in that** antibiotics which are freely soluble in water are optionally used as organic composite components.

17. Process for the preparation of antibiotic composites according to any of Claims 1 to 16, **characterized in that** the mass fraction of the plastically deformable salt in the composites is from 0.1 to 98 percent by mass.

18. Process for the preparation of antibiotic composites according to any of Claims 1 to 17, **characterized in that** the composites are shaped to give mouldings, granules and powders.

19. Process for the preparation of antibiotic composites according to any of Claims 1 to 18, **characterized in that** the composites are plastically deformable.

20. Process for the preparation of antibiotic composites according to any of Claims 1 to 17 or 19, **characterized in that** the composites are used in the form of pastes.

21. Process for the preparation of antibiotic composites according to any of Claims 1 to 20, **characterized in that** the composites are applied as a coating to resorbable implants and to nonresorbable implants.

## Revendications

1. Procédé de préparation de composites antibiotiques, **caractérisé en ce que** l'on utilise, à titre de liant, le cas échéant en additionnant de l'eau pour le formage des composites, un sel déformable plastiquement qui est constitué d'au moins un composant cationique d'une base antibiotique protonée, choisi dans les groupes d'antibiotiques de la famille des aminoglycosides, d'antibiotiques de la famille des lincosamides et d'antibiotiques de la famille des tétracyclines et d'au moins un composant anionique choisi dans le groupe des carboxylates aliphatiques, alkylsulfates, arylsulfates, alkylarylsulfates, cycloalkylsulfates, alkylcycloalkylsulfates, allcylsulfamates, cycloalkylsulfamates, allcylcycloalkylsulfamates, arylsulfamates, alkylarylsulfamates, alkylsulfonates, 2-sulfonates d'acides gras, arylsulfonates, alkylarylsulfonates, cycloalkylsulfonates, alkylcycloalkylsulfonates, alkyldisulfates, cycloalkyldisulfates, alkyldisulfonates, cycloalkyldisulfonates, aryldisulfonates, alkylaryldisulfonates, aryltrisulfonates et alkylaryltrisulfonates.

2. Procédé de préparation de composites antibiotiques selon la revendication 1, **caractérisé en ce que** le formage est opéré par pressage et/ou boudinage et/ou laminage et/ou calandrage et/ou broyage.

3. Procédé de préparation de composites antibiotiques selon l'une des revendications 1 à 2, **caractérisé en ce que** l'on utilise, à titre de composant anionique choisi dans le groupe des alkylsulfates, en particulier le dodécylsulfate, le tétradécylsulfate, fhexadécylsulfate, l'octadécylsuliate et le docosanolsulfate.

4. Procédé de préparation de composites antibiotiques selon l'une des revendications 1 à 2, **caractérisé en ce que** l'on utilise, à titre de composant anionique choisi dans le groupe des alkylsulfonates, le dodécylsulfonate, l'hexadécylsulfonate et l'octadécylsulfonate.

5. Procédé de préparation de composites antibiotiques selon l'une des revendications 1 à 2, **caractérisé en ce que** l'on utilise, à titre de composant anionique, en particulier des carboxylates aliphatiques comprenant de 12 à 30 atomes de carbone.

6. , Procédé de préparation de composites antibiotiques selon l'une des revendications 1 à 2, **caractérisé en ce que** l'on utilise, à titre de composant anionique choisi dans le groupe des carboxylates aliphatiques, le palmitate, le stéarate et le béhénylate.

7. Procédé de préparation de composites antibiotiques selon l'une des revendications 1 à 2, **caractérisé en ce que** l'on synthétise le sel déformable plastiquement avant le procédé de formage.

8. Procédé de préparation de composites antibiotiques selon la revendication 1, **caractérisé en ce que** l'on forme le sel déformable plastiquement pendant le formage des composites en introduisant de l'eau dans un mélange constitué d'ingrédients composites inorganiques, le cas échéant d'ingrédients composites organiques, d'un ou de plusieurs représentants des antibiotiques de la famille des aminoglycosides et/ou des antibiotiques de la famille des lincosamides et/ou des antibiotiques de la famille des tétracyclines, qui se présentent sous forme de sulfate, chlorhydrate, bromhydrate et phosphate, et constitué d'un ou de plusieurs représentants des alkylsulfates, arylsulfates, alkylarylsulfates, cycloalkylsulfates, alkylcycloalkylsulfates, alkylsulfamates, cycloalkylsulfamates, alkylcycloalkylsulfamates, arylsulfamates, alkylarylsulfamates, alkylsulfonates, 2-sulfonates d'acides gras, arylsulfonates, alkylarylsulfonates, cycloalkylsulfonates, alkylcycloalkylsulfonates, alkyldisulfates, cycloalkyldisulfates, alkyldisulfonates, cycloalkyldisulfonates, aryldisulfonates, alkylaryldisulfonates, aryltrisulfonates et alkylaryltrisulfonates, qui se présentent sous forme de sel sodique et/ou sous forme de sel potassique et/ou sous forme de sel d'ammonium et/ou sous forme de sel de trialkylammonium et/ou sous forme de sel de dialkylammonium et/ou sous forme de sel de monoalkylammonium et/ou sous forme de sel de triarylammonium et/ou sous forme de sel de diarylammonium et/ou sous forme de sel d'arylammonium et/ou sous forme de sel d'alkyldiarylammonium et/ou sous forme de sel de dialkylarylammonium et/ou sous forme de sel de tricycloalkylammonium et/ou sous forme de sel de dicycloalkylammonium et/ou sous forme de sel de monocycloalkylammonium et/ou sous forme de sel d'alkyldicycloalkylammonium et/ou sous forme de sel de dialkylcycloalkylammonium.

9. Procédé de préparation de composites antibiotiques selon l'une des revendications 1 à 2 ou 8, **caractérisé en ce que** les alkylsulfates, arylsulfates, alkylarylsulfates, cycloalkylsulfates et aikylcycloalkylsulfates sont des hémiesters d'acide sulfurique.

10. Procédé de préparation de composites antibiotiques selon l'une des revendications 1, 2, 8 ou 9, **caractérisé en ce que** l'on utilise, à titre d'alkylsulfates, le dodécylsulfate de sodium, le tétradécylsulfate de sodium, l'hexadécylsulfate de sodium et l'octadécylsulfate de sodium.

11. Procédé de préparation de composites antibiotiques selon l'une des revendications 1 à 2 ou 8, **caractérisé en ce que** l'on utilise, à titre d'alkylsulfonates, le dodécylsulfonate de sodium, l'hexadécylsulfonate de sodium et l'octadécylsulfonate de sodium.

12. Procédé de préparation de composites antibiotiques selon l'une des revendications 1 à 2 ou 8, **caractérisé en ce que** l'on utilise, à titre d'alkylarylsulfonate, en particulier le dodécylbenzylsulfonate de sodium.

13. Procédé de préparation de composites antibiotiques selon l'une des revendications 1 à 12, **caractérisé en ce que** l'on utilise, à titre d'ingrédients composites inorganiques, le carbonate de calcium, carbonate de magnésium, hydroxyde de calcium, hydroxyde de magaésium, oxyde de magnésium, sulfate de calcium, sulfate de calcium hémihydraté, sulfate de calcium dihydraté, phosphate tricalcique, phosphate tétracalcique, hydrogénophosphate de calcium, hydrogénophosphate de calcium dihydraté, hydroxylapatite, fluorapatite, verre résorbable, céramique de verre résorbable et leurs mélanges.

14. Procédé de préparation de composites antibiotiques selon l'une des revendications 1 à 13, **caractérisé en ce que** l'on utilise les ingrédients composites inorganiques sous forme de poudres et/ou de granulés.

15. Procédé de préparation de composites antibiotiques selon l'une des revendications 1 à 14, **caractérisé en ce que** l'on utilise à titre d'ingrédients composites organiques de préférence l'amidon, la cellulose, la quitine, le chitosane, la gélatine, le collagène, les esters d'acide polyméthacrylique, les esters d'acide polyacrylique, l'alcool polyvinylique, le chlorure de polyvinyle, le chlorure de polyvinylidène, le polytétrafluométhylène, la cire d'abeilles, la cire de carnauba, les triglycérides et leurs mélanges.

16. Procédé de préparation de composites antibiotiques selon l'une des revendications 1 à 15, **caractérisé en ce que** l'on utilise des antibiotiques le cas échéant facilement solubles dans l'eau à titre de composants composites organiques.

17. Procédé de préparation de composites antibiotiques selon l'une des revendications 1 à 16, **caractérisé en ce que** la proportion en poids du sel déformable plastiquement dans les composites atteint 0,1 à 98 % en poids.

18. Procédé de préparation de composites antibiotiques selon l'une des revendications 1 à 17, **caractérisé en ce que** les composites sont façonnés en corps moulés, granulés et poudres.

19. Procédé de préparation de composites antibiotiques selon l'une des revendications 1 à 18, **caractérisé en ce que** les composites sont déformables plastiquement.

20. Procédé de préparation de composites antibiotiques selon l'une des revendications 1 à 17 ou 19, **caractérisé en ce que** les composites sont utilisés sous forme de pâtes.

21. Procédé de préparation de composites antibiotiques selon l'une des revendications 1 à 20, **caractérisé en ce que** les composites sont déposés à titre de revêtement sur des implants résorbables et sur des implants non résorbables.
